# EUROPEAN PATENT APPLICATION

(11) **EP 3 824 803 A1**
(43) Date of publication of application: **26.05.2021**
(21) Application number: 19838546.0
(22) Date of filing: 20.06.2019
(51) Int. Cl.: A61B 5/026

(54) **MEASURING DEVICE AND MEASUREMENT METHOD**

(30) Priority: 18.07.2018 JP 2018134986
(71) Applicant: Sony Corporation, 108-0075 Tokyo (JP)
(72) Inventor: MIYASHITA, Ken, Tokyo 108-0075 (JP); SATO, Makoto, Tokyo 108-0075 (JP)
(74) Representative: 2SPL Patentanwälte PartG mbB
(86) International application number: PCT/JP2019/024554
(87) International publication number: WO 2020/017231

(57) **Abstract**

A measuring device provided with a pressing unit that presses a part of a user's body according to adjustment by a user, a pressure measuring unit that measures a pressing force by the pressing unit, and a blood flow measuring unit that measures a blood flow velocity of the user in a case where the pressing force is in a predetermined state.

## Description

### TECHNICAL FIELD

The present technology relates to a measuring device and a measuring method.

### BACKGROUND ART

Conventionally, a measuring device for measuring a blood pressure capable of easily measuring the blood pressure and is convenient is suggested (Patent Document 1) .

### CITATION LIST

### PATENT DOCUMENT

Patent Document 1: Japanese Patent Application Laid-Open No. 2014-12072

### SUMMARY OF THE INVENTION

### PROBLEMS TO BE SOLVED BY THE INVENTION

Such measuring device is required to be convenient and to measure a blood pressure with high accuracy. In order to measure a blood pressure with high accuracy, a method referred to as an oscillometric method is conventionally effective.

Since the oscillometric method usually requires a cuff and a large air pump, a device becomes large in size, and this is not suitable for the purpose of carrying the same and easily measuring a blood pressure. Furthermore, it is difficult to easily measure the blood pressure in daily life because the measurement using the cuff or air pump takes time and effort.

The present technology is achieved in view of such circumstances, and an object thereof is to provide a measuring device and a measuring method capable of easily measuring a blood pressure with a simple configuration.

### SOLUTIONS TO PROBLEMS

In order to solve the above-mentioned problem, a first technology is a measuring device provided with a pressing unit that presses a part of a user's body according to adjustment by a user, a pressure measuring unit that measures a pressing force by the pressing unit, and a blood flow measuring unit that measures a blood flow velocity of the user in a case where the pressing force is in a predetermined state.

Furthermore, a second technology is a measuring method provided with pressing a part of a user's body by a pressing unit according to adjustment by a user, measuring a pressing force by the pressing unit, and measuring a blood flow velocity of the user in a case where the pressing force is in a predetermined state.

Furthermore, a third technology is a measuring device provided with a pressing unit that presses a part of a user's body, a pressure measuring unit that measures a pressing force by the pressing unit, a blood flow measuring unit that measures a blood flow velocity of a user in a case where the pressing force is in a predetermined state, a target pressure setting unit that sets a target pressure being a target of the pressing force, and a pressing control unit that controls the pressing unit such that the pressing force matches the target pressure.

Furthermore, a fourth technology is a measuring method provided with pressing a part of a user's body to measure a pressing force, measuring a blood flow velocity of a user in a case where the pressing force is in a predetermined state, setting a target pressure being a target of the pressing force, and controlling the pressing unit such that the pressing force matches the target pressure.

### EFFECTS OF THE INVENTION

According to the present technology, a blood pressure may be easily measured with a simple configuration. Note that, the effects are not necessarily limited to the effects herein described and may be any effect described in the present specification.

### BRIEF DESCRIPTION OF DRAWINGS

Fig. 1 is a block diagram illustrating a configuration of a measuring device according to a first embodiment.
Fig. 2 is an external view of the measuring device.
Fig. 3 is a view illustrating a configuration of a pressing unit according to the first embodiment.
Fig. 4 is an explanatory view of a blood pressure measuring method.
Fig. 5 is a flowchart illustrating an instruction information outputting process until a maximum blood pressure is measured.
Fig. 6 is a flowchart illustrating an instruction information outputting process until a minimum blood pressure is measured.
Fig. 7 is an explanatory view of a user interface of the measuring device.
Fig. 8 is an explanatory view of an output of instruction information.
Fig. 9 is an explanatory view of an output of instruction information.
Fig. 10 is an explanatory view of an output of instruction information.
Fig. 11 is a block diagram illustrating a configuration of an information processing device.
Fig. 12 is a block diagram illustrating a configuration of an information processing device and a terminal device.
Fig. 13 is a block diagram illustrating a configuration of a measuring device according to a second embodiment.
Fig. 14 is a view illustrating a configuration of a pressing unit.
Fig. 15 is a flowchart illustrating a pressing controlling process until a maximum blood pressure is measured.
Fig. 16 is a flowchart illustrating a pressing controlling process until a minimum blood pressure is measured.
Fig. 17 is a view illustrating a variation of a measuring device.

### MODE FOR CARRYING OUT THE INVENTION

An embodiment of the present technology is hereinafter described with reference to the drawings. Note that, the description is given in the following order.
<1. First Embodiment>
[1-1. Configuration of measuring device]
[1-2. Configuration of pressing unit]
[1-3. Blood pressure measuring method]
[1-4. Instruction information outputting process]
[1-5. User interface]
<2. Second Embodiment>
[2-1. Configuration of measuring device]
[2-2. Configuration of pressing unit]
[2-3. Pressing controlling process]
<3. Variation>

### <1. First Embodiment>

### [1-1. Configuration of measuring device]

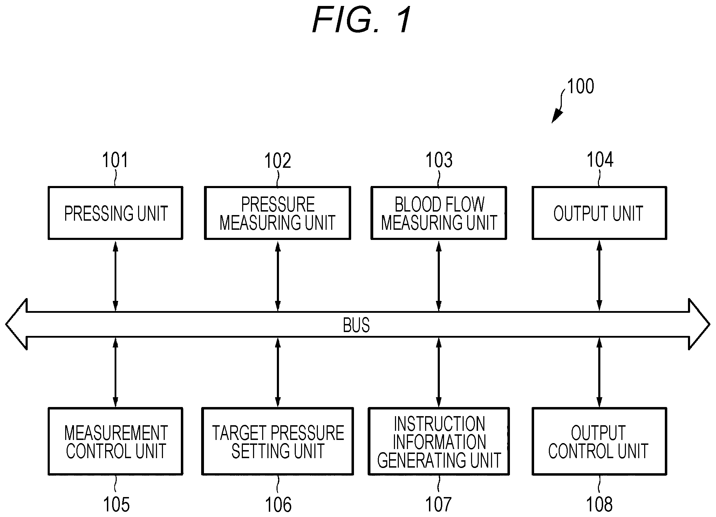

First, a configuration of a measuring device 100 is described with reference to Fig. 1. The measuring device 100 is provided with a pressing unit 101, a pressure measuring unit 102, a blood flow measuring unit 103, an output unit 104, a measurement control unit 105, a target pressure setting unit 106, an instruction information generating unit 107, and an output control unit 108.

The pressing unit 101 is a pressing mechanism that presses a part of a user's body (for example, a wrist in a case where the measuring device 100 is worn on the wrist) by a pressing force based on adjustment by a user.

The pressure measuring unit 102 is a known pressure sensor that measures the pressing force on a part of the user's body by the pressing unit 101.

The blood flow measuring unit 103 is a blood flow sensor that measures a blood flow velocity in a state in which the pressing force and a target pressure match. A change in a blood flow velocity over time is measured as a pulse wave. As a method of measuring the pulse wave, there are a photoplethysmography (PPG) method, a pressure sensor method, and the like. The PPG method is a method in which light is applied to a blood vessel and reflected light is detected by a light detector, and a change in light absorption amount in association with a change in capacity of the blood vessel occurring with deliver of blood is obtained as the pulse wave. The pressure sensor method is a method in which a piezoelectric pressure wave sensor module including a sensor block and a control substrate is used to detect a minute pressure change or oscillation with high sensitivity, thereby detecting the pulse wave. As a method of measuring the blood flow velocity, there are a laser Doppler method, an optical fiber method, an ultrasonic method, and the like. The laser Doppler method measures the blood flow velocity based on scattered light intensity fluctuation information from living tissue. The optical fiber method is a method of applying and receiving a laser beam to and from the living tissue by means of an optical fiber, and measuring the blood flow velocity based on the scattered light intensity fluctuation information from the living tissue. The ultrasonic method is a method of measuring the blood flow velocity using an ultrasonic Doppler method.

The output unit 104 is a display monitor including a liquid crystal display (LCD), a plasma display panel (PDP), an organic electro luminescence (EL) panel, and the like, for example. The output unit 104 displays a user interface of the measuring device 100, an instruction of pressing force adjustment, and the like. Note that, the output unit 104 may be a speaker that outputs a sound.

The measurement control unit 105 controls an entire measuring device 100 and each unit. The measurement control unit 105 also performs measurement control so that the blood flow measuring unit 103 measures the blood flow velocity of the user in a state in which the pressing force measured by the pressure measuring unit 102 matches the target pressure. Furthermore, this also performs a process of obtaining a blood pressure of the user on the basis of the pressing force measured by the pressure measuring unit 102 and the blood flow velocity measured by the blood flow measuring unit 103. The pressing force at a time when the pulse wave that is a change in blood flow velocity over time disappears is obtained as a maximum blood pressure. Furthermore, the pressing force that compresses the user's body when the pressing force is reduced from the state in which the pulse wave disappears and steady amplitude of the pulse wave is restored is obtained as a minimum blood pressure.

The target pressure setting unit 106 sets the target pressure serving as a target of the pressing force by the pressing unit 101. The measuring device 100 presents instruction information to the user so that the target pressure and the pressing force by the pressing unit 101 match to issue an adjustment instruction of the pressing force. Note that, when the target pressure matches the pressing force of the pressing unit 101, they may be completely the same or may approximate within a predetermined range. The target pressure setting unit 106 checks amplitude of the pulse wave that is the change in blood flow velocity measured by the blood flow measuring unit 103 over time, and updates the target pressure so as to gradually increase the pressing force until the maximum blood pressure is measured on the basis of an oscillometric method, and updates the target pressure so as to gradually decrease the pressing force after the maximum blood pressure is measured.

The instruction information generating unit 107 compares the target pressure set by the target pressure setting unit 106 with the current pressing force by the pressing unit 101 measured by the pressure measuring unit 102 and generates the instruction information to be presented to the user (pressing adjustment instruction).

The output control unit 108 controls the output unit 104 to output the instruction information supplied from the instruction information generating unit 107.

The measuring device 100 is configured as described above. According to a procedure of the oscillometric method, the measuring device 100 gradually increases the pressing force by the pressing unit 101, and when the pulse wave disappears and the maximum blood pressure may be measured, this gradually decreases the pressing force by the pressing unit 101 to measure the minimum blood pressure.

The measurement control unit 105, the target pressure setting unit 106, the instruction information generating unit 107, and the output control unit 108 are configured by a program, and the program may be installed on the measuring device 100 in advance, or may be distributed by downloading, by a storage medium and the like to be installed on the measuring device 100 by the user. Furthermore, the measurement control unit 105, the target pressure setting unit 106, the instruction information generating unit 107, and the output control unit 108 may be realized not only by a program but also by combination of a dedicated device with hardware having this function, a circuit, and the like.

### [1-2. Configuration of pressing unit]

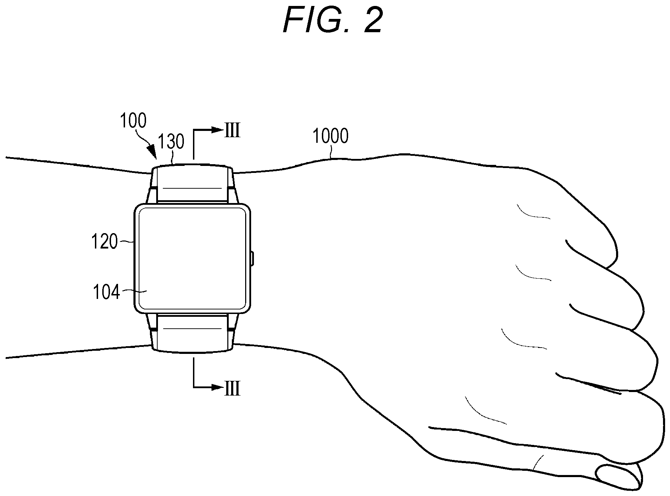

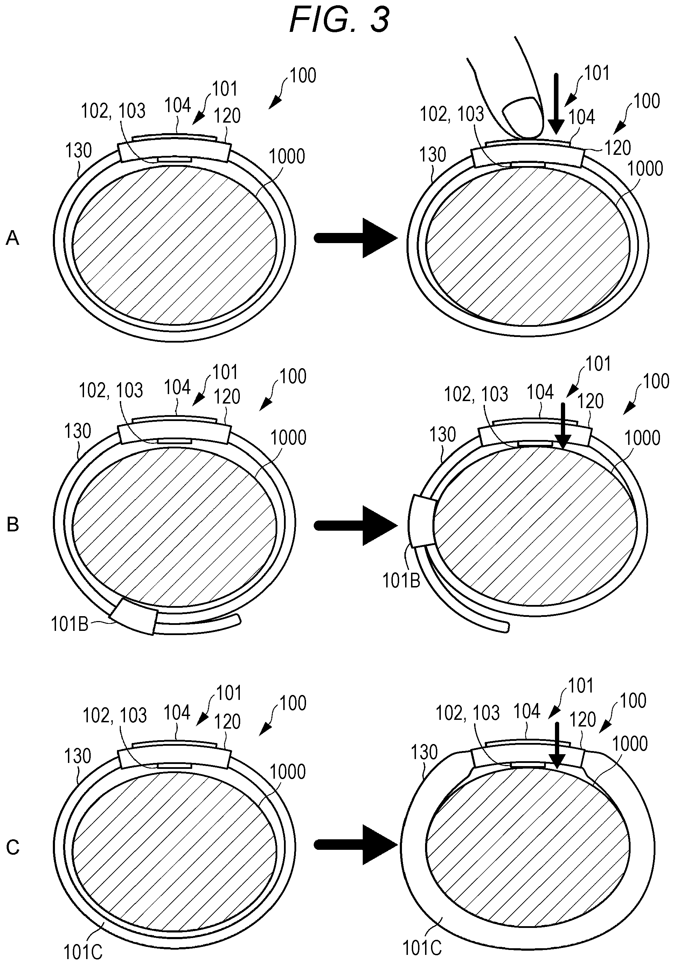

Next, a configuration of the pressing unit 101 is described. Note that, configuration examples of the pressing unit 101 include first to third examples, and in any example, the measuring device 100 includes a casing 120 and a band portion 130 and is a wearable device wound around a user's arm 1000 as illustrated in Fig. 2. Furthermore, in any of the first to third examples, as illustrated in Fig. 3, the pressure measuring unit 102 and the blood flow measuring unit 103 are arranged close to each other on a side brought into contact with the user's arm 1000 on an inner side of the measuring device 100. This is because the blood flow velocity is measured in a position where the pressing force is applied and the blood flow of the user changes.

Fig. 3 illustrating the configuration of the pressing unit 101 is a cross-sectional view taken along line III-III in Fig. 2. In the first example, as illustrated in Fig. 3A, the casing 120 itself serves as the pressing unit 101 and the casing 120 is pressed from a side opposite to a surface on which the pressure measuring unit 102 and the blood flow measuring unit 103 are provided in a direction toward the user's arm 1000. Therefore, the casing 120 and the pressure measuring unit 102 may press the user's arm 1000 to eliminate the pulse wave, and the blood flow measuring unit 103 may measure the blood flow velocity in a pressed site.

In the second example, the pressing unit 101 is configured as an adjusting mechanism 101B that adjusts a length of the band portion 130 of the measuring device 100 as illustrated in Fig. 3B. By adjusting a tightening degree of the band portion 130 with this adjusting mechanism 101B, the casing 120 and the pressure measuring unit 102 may press the user's arm 1000 to eliminate the pulse wave, and the blood flow measuring unit 103 may measure the blood flow velocity in the pressed site. The adjusting mechanism 101B may be of any type such as a slide type, a pin type, and a screw type as long as the tightening degree may be adjusted by a user operation.

In the third example, as illustrated in Fig. 3C, the pressing unit 101 is configured as an adjusting mechanism 101C that adjusts the tightening degree of the arm 1000 by the band portion 130 by adjusting a diameter (thickness) of the band portion 130. By adjusting the tightening degree of the band portion 130 by this adjusting mechanism 101C, the casing 120 and the pressure measuring unit 102 may press the user's arm 1000 to eliminate the pulse wave, and the blood flow measuring unit 103 may measure the blood flow velocity at the pressed site. As a method of adjusting the diameter (thickness) of the band portion 130, for example, there is a method in which the inside of the band portion 130 is made a highly airtight cavity and the user supplies air to the inside of the band portion 130 with a pump to inflate.

### [1-3. Blood pressure measuring method]

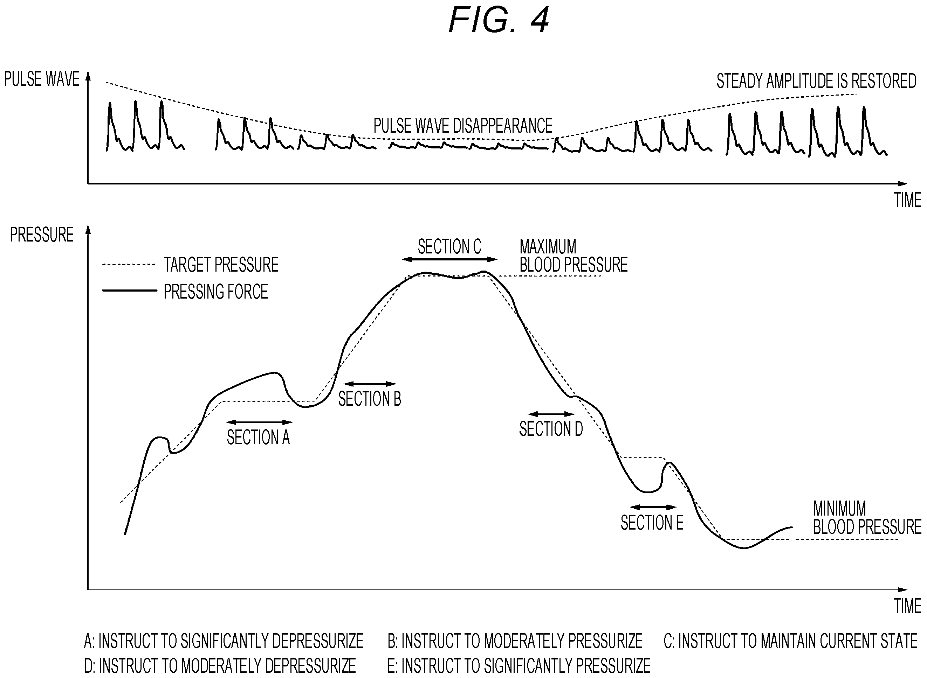

Next, an outline of blood pressure measurement according to the present technology is described with reference to Fig. 4. The present technology measures the blood pressure on the basis of the oscillometric method. Specifically, by compressing a part of the user's body such as the wrist by pressing and measuring the blood flow velocity of a compressed part, the blood pressure is measured on the basis of the change in pulse wave being the change in blood flow over time.

As illustrated in Fig. 4, the pressing force to compress the user's body at the time when the pulse wave disappears is the maximum blood pressure, and the pressing force to compress the user's body at the time when it returns from the state in which the pulse wave disappears to a steady amplitude state is the minimum blood pressure.

Therefore, the present technology checks the amplitude of the pulse wave while measuring the blood flow velocity by the blood flow measuring unit 103, and gradually increases the pressing force until the pulse wave disappears. At that time, the target pressure is set for pressurization, and the user is instructed by the instruction information to perform the pressing by the pressing unit 101 at the target pressure. Then, the user increases the pressing force to match the target pressure on the basis of the instruction information, and the pressing force at the time when the pulse wave disappears is set as the maximum blood pressure.

After the maximum blood pressure is measured, the target pressure is set for depressurization, and the user is instructed by the instruction information to perform the pressing by the pressing unit 101 at the target pressure. Then, the user performs the pressing at the target pressure on the basis of the instruction information to gradually decrease the pressing force, and the pressing force at the time when the steady amplitude of the pulse wave is restored is set as the minimum blood pressure. The steady amplitude is the amplitude of the pulse wave in a normal state in which no load is applied by the pressing.

A graph illustrated in Fig. 4 is merely an example presented for convenience of explanation, but in a case of the graph in Fig. 4, the pressing force is much larger than the target pressure in a section A, so that the instruction information is output to significantly decrease the pressing force (depressurize). In a section B, the target pressure continuously increases, so that the instruction information is output to increase the pressing force (pressurize) modestly so as to match the same. Since the target pressure is constant in a section C, in order to maintain the pressing force that matches the target pressure, the instruction information is output to maintain the current pressing force.

In a section D, since the target pressure continuously decreases, the instruction information is output so as to decrease the pressing force (depressurize) modestly so as to match the same. Since the pressing force is much smaller than the target pressure in a section E, the instruction information is output so as to significantly increase the pressing force (pressurize).

In this manner, by allowing the user to appropriately adjust the pressing force by the instruction information, it becomes possible to easily measure the blood flow velocity and measure the blood pressure.

### [1-4. Instruction information outputting process]

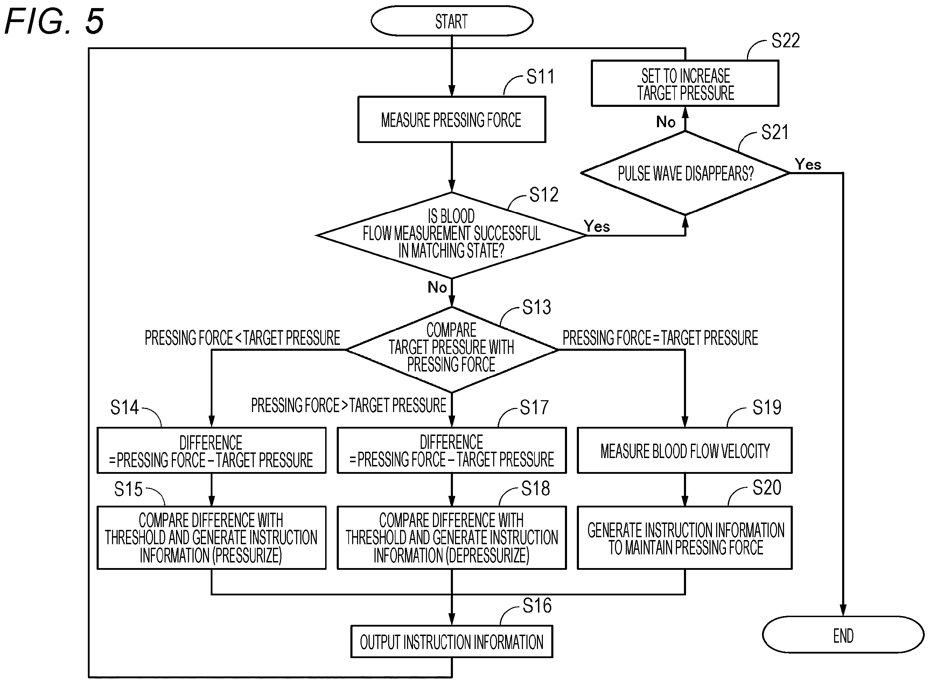

Next, an instruction information outputting process for adjusting the pressing force until the maximum blood pressure is measured is described with reference to a flowchart in Fig. 5.

First, at step S11, the pressure measuring unit 102 measures the pressing force by the pressing unit 101. Next, at step S12, the measurement control unit 105 checks whether or not the blood flow measuring unit 103 successfully measures the blood flow velocity in a matching state of the pressing force and the target pressure.

In a case where the blood flow is not successfully measured at the target pressure in the matching state of the pressing force and the target pressure, the procedure shifts to step S13 (No at step S12). Next, at step S13, the instruction information generating unit 107 compares the target pressure with the current pressing force measured by the pressure measuring unit 102 at step S11.

As a result of the comparison, in a case where the pressing force is smaller than the target pressure, the procedure shifts to step S14, and the instruction information generating unit 107 calculates a difference between the pressing force and the target pressure. Next, at step S15, the instruction information generating unit 107 generates instruction information according to the difference as the instruction information.

In the generation of the instruction information at step S15, the difference between the pressing force and the target pressure calculated at step S14 is compared with a predetermined threshold, and in a case where the difference is larger than the predetermined threshold, the instruction information is made an "instruction to significantly pressurize". On the other hand, in a case where the difference between the pressing force and the target pressure is smaller than the predetermined threshold, the instruction information is made an "instruction to modestly pressurize". By setting a degree of the pressurizing instruction in this manner and providing steps in the pressurizing instruction, it becomes possible to adjust the pressing force more finely.

Then, at step S16, the output control unit 108 allows the output unit 104 to output the instruction information to be presented to the user. The user adjusts the pressing force with reference to the instruction information being the pressurizing instruction.

The description returns to step S13. As a result of the comparison at step S13, in a case where the pressing force is larger than the target pressure, the procedure shifts to step S17, and the instruction information generating unit 107 calculates a difference between the pressing force and the target pressure. Next, at step S18, the instruction information generating unit 107 generates instruction information according to the difference as the instruction information.

In the generation of the instruction information at step S18, the difference between the pressing force and the target pressure calculated at step S17 is compared with a predetermined threshold, and in a case where this is larger than the predetermined threshold, the instruction information is made an "instruction to significantly depressurize". On the other hand, in a case where the difference between the pressing force and the target pressure is smaller than the predetermined threshold, the instruction information is made an "instruction to modestly depressurize". By setting a degree of the depressurizing instruction in this manner and providing steps in the depressurizing instruction, it becomes possible to adjust the pressing force more finely.

Then, at step S16, the output control unit 108 allows the output unit 104 to output the instruction information to be presented to the user. The user adjusts the pressure with reference to the instruction information being the depressurizing instruction.

The description returns to step S13. As a result of the comparison at step S13, in a case where the pressing force and the target pressure match, the procedure shifts to step S19, and the blood flow measuring unit 103 measures the blood flow velocity of the user. Next, at step S20, the instruction information generating unit 107 generates an instruction to maintain the pressing force, and at step S16, the output control unit 108 controls the output unit 104 to present the instruction information to maintain the pressing force to the user.

The description returns to step S12. In a case where the blood flow velocity is successfully measured in the matching state of the pressing force and the target pressure at step S12, the procedure shifts to step S21 (Yes at step S12). Then, at step 21, it is determined whether or not the pulse wave disappears. In a case where the pulse wave does not disappear, the procedure shifts to step S22 (No at step S21). It is possible to determine whether or not the pulse wave disappears by checking the amplitude of the pulse wave that is a change in blood flow in blood flow velocity over time.

Then, at step S22, the target pressure setting unit 106 sets a new target pressure higher than the current target pressure. Note that, in a case where the target pressure is set by the target pressure setting unit 106, it is preferable to set an upper limit value in advance such that the target pressure is not set higher than the upper limit value. This is a safety measure to prevent the target pressure from being set to an abnormally high value so that the user's body is suddenly pressed with a strong pressing force. Next, the procedure returns to step S11, and thereafter, a process is performed on the basis of the new target pressure set at step S22.

Then, in a case where the pulse wave disappears at step S21, the procedure ends (Yes at step S21). This is because the maximum blood pressure is the pressing force when the pulse wave disappears, so that it becomes not necessary to output the instruction information for increasing the pressing force when pulse wave disappearance disappears.

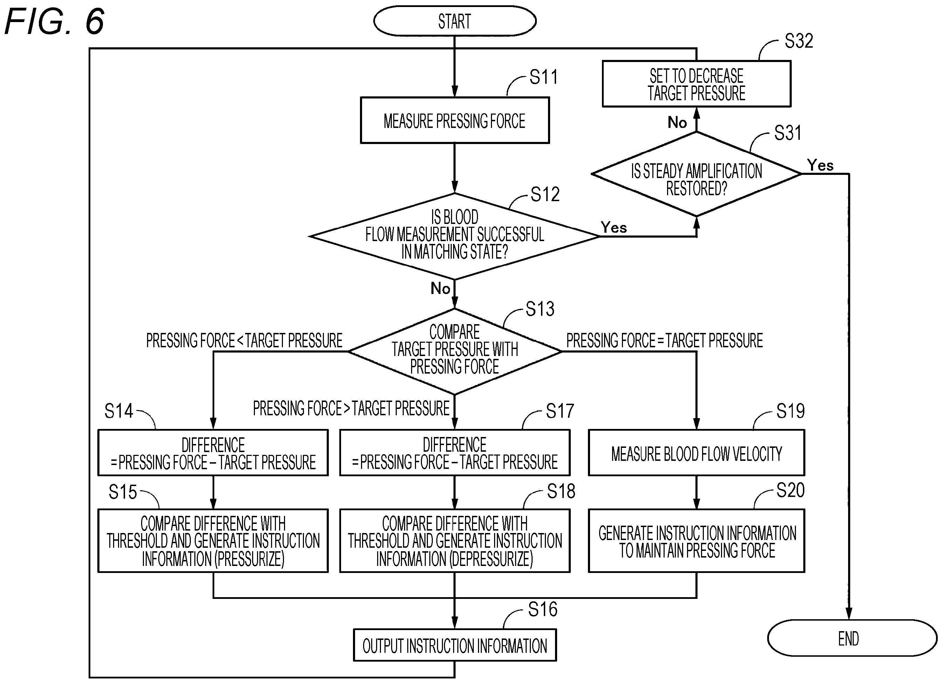

Next, an instruction information outputting process for adjusting the pressing force until the minimum blood pressure is obtained is described with reference to a flowchart in Fig. 6. Note that, steps S11 to S20 of processes similar to those in the flowchart in Fig. 5 are not described.

In a case where the blood flow velocity is successfully measured in the matching state of the pressing force and the target pressure at step S12, the procedure shifts to step S31 (Yes at step S12). Then, at step 31, it is determined whether or not the steady amplitude of the pulse wave is restored. In a case where the steady amplitude of the pulse wave is not restored, the procedure shifts to step S32 (Yes at step S31).

Then, at step S32, the target pressure setting unit 106 sets a new target pressure lower than the current target pressure. Next, the procedure returns to step S11, and thereafter, a process is performed on the basis of the new target pressure set at step S32.

Then, in a case where the steady amplitude of the pulse wave is restored at step S31, the procedure ends (Yes at step S31). This is because the minimum blood pressure is the pressing force when the steady amplitude of the pulse wave is restored, so that it becomes not necessary to output the instruction information for decreasing the pressing force when the steady amplitude is restored.

The instruction information outputting process is performed as described above.

### [1-5. User interface]

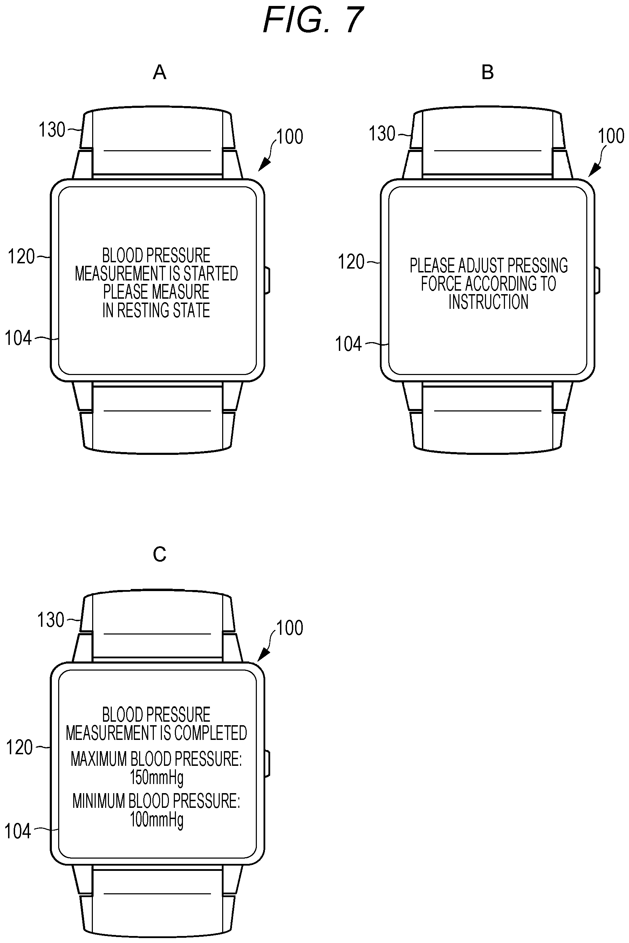

Next, with reference to Figs. 7 to 10, a user interface and the instruction information output by the output unit 104 of the measuring device 100 are described. Figs. 7 to 10 illustrate examples in a case where the output unit 104 is a display.

First, when the blood pressure measurement by the measuring device 100 is started, a message indicating that the blood pressure measurement is started is output by the output unit 104 as illustrated in Fig. 7A. Next, after a lapse of a fixed time from the output of the message illustrated in Fig. 7A, a message indicating a method of measuring the blood pressure is output by the output unit 104 as illustrated in Fig. 7B.

Then, when the blood pressure measurement is finished, the output unit 104 outputs a message indicating that the blood pressure measurement is finished and the maximum blood pressure and the minimum blood pressure as illustrated in Fig. 7C. This allows the user to know his/her blood pressure measurement result. Note that, the user interface during the blood pressure measurement is described later.

In a case where the output unit 104 is a speaker, a message similar to that illustrated in Fig. 7 may be output by a sound. Furthermore, a display on the display and a sound output from the speaker may be performed at the same time.

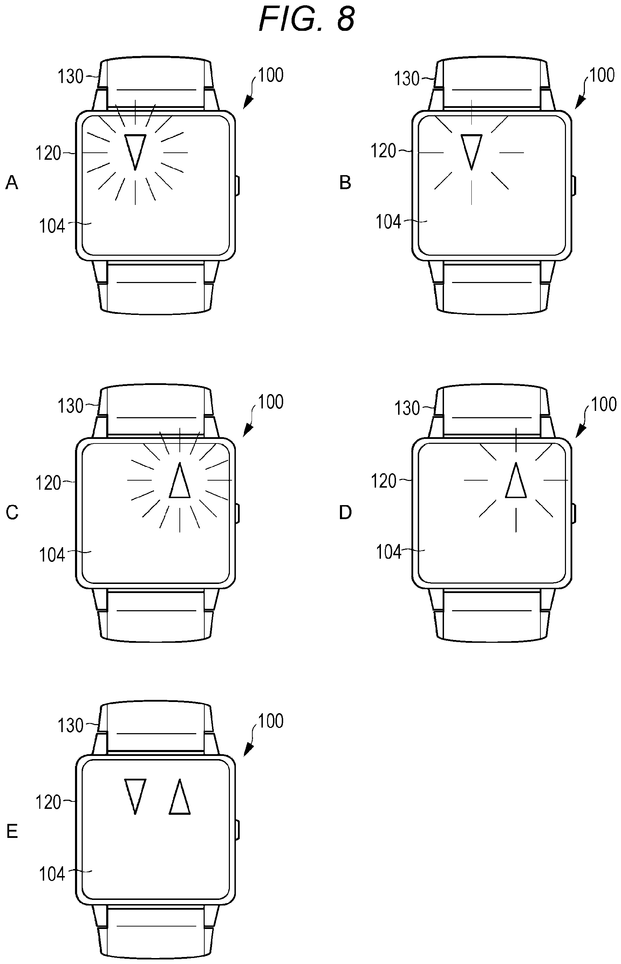

Next, with reference to Figs. 8 to 10, the output of the instruction information for adjusting the pressing force is described. In the present technology, when the user is urged to adjust the pressing force, not the instruction presenting a specific numerical value but the instruction information with which the user may understand to "make the pressing force stronger than the current pressing force" or to "make the pressing force weaker than the current pressing force" is output. This allows the user to intuitively adjust the pressing force.

In a case where the output unit 104 is the display, there are various instructing methods as illustrated in Figs. 8 to 10. First, blinking of light illustrated in Fig. 8 is described.

As illustrated in Figs. 8A and 8B, an instruction to pressurize is issued by blinking a down-pointing icon. Since the pressurization by the adjustment by the user is to push the casing 120, the pressure measuring unit 102, and the blood flow measuring unit 103 in a direction toward the user's body, presentation of the down-pointing icon may allow the user to intuitively understand that this is an instruction to pressurize.

In a case of the instruction to significantly pressurize from the current pressing force, the down-pointing icon blinks rapidly as illustrated in Fig. 8A. On the other hand, in a case of the instruction to modestly pressurize from the current pressing force, the down-pointing icon blinks slower than that at the time of the significant pressurization as illustrated in Fig. 7B. This allows the user to intuitively understand a pressurization degree.

On the other hand, an instruction to depressurize is issued by blinking an up-pointing icon as illustrated in Figs. 8C and 8D. Since the depressurization by the user operation is to weaken the pressing force to push the measuring device 100 in the direction toward the user's body, presentation of the up-pointing icon may allow the user to intuitively understand that this is an instruction to depressurize.

In a case of the instruction to significantly depressurize from the current pressing force, the up-pointing icon blinks rapidly as illustrated in Fig. 8C. On the other hand, in a case of the instruction to modestly depressurize from the current pressing force, the up-pointing icon blinks slower than that at the time of the significant depressurization as illustrated in Fig. 7D. This allows the user to intuitively understand a depressurization degree.

In a case of the instruction to maintain the current pressing force, both the up-pointing icon and the down-pointing icon are turned on as illustrated in Fig. 8E. Therefore, the user may intuitively understand that the instruction is not to pressurize or depressurize but to maintain the pressing force.

Note that, a blinking speed of the icon is, for example, one blink per 0.5 seconds in a case of the significant pressurization/depressurization, and one blink per 1 second in a case of the modest pressurization/depressurization.

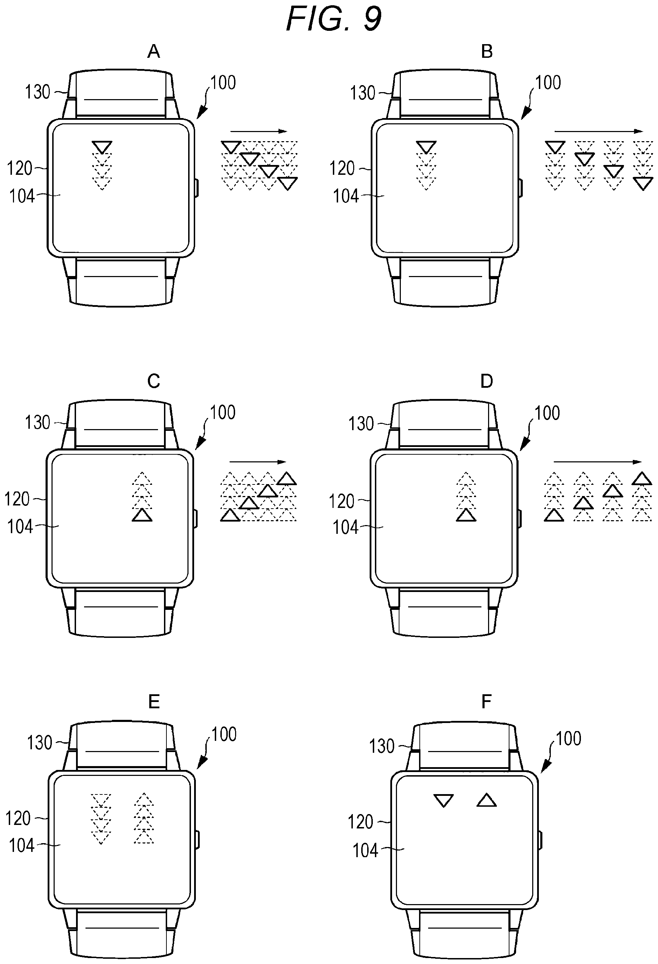

Next, an animation illustrated in Fig. 9 is described. Note that, in Figs. 9A, 9B, 9C, and 9D, a drawing on a right side of the measuring device 100 illustrates movement of an icon by the animation.

As illustrated in Figs. 9A and 9B, an instruction to pressurize is issued by moving the icon downward. Since the pressurization by the user operation is to push the measuring device 100 in the direction toward the user's body, movement of the icon downward may allow the user to intuitively understand that this is an instruction to pressurize.

In a case of the instruction to significantly pressurize from the current pressing force, the icon is rapidly moved downward as illustrated in Fig. 9A. On the other hand, in a case of the instruction to modestly pressurize from the current pressing force, the icon is moved downward slower than that at the time of the significant pressurization as illustrated in Fig. 8B. This allows the user to intuitively understand a pressurization degree.

On the other hand, an instruction to depressurize is issued by moving the icon upward as illustrated in Figs. 9C and 9D. Since the pressurization by the user operation is to push the measuring device 100 in the direction toward the user's body, movement of the icon upward may allow the user to intuitively understand that this is an instruction to depressurize.

In a case of the instruction to significantly depressurize from the current pressing force, the icon is rapidly moved upward as illustrated in Fig. 9C. On the other hand, in a case of the instruction to modestly depressurize from the current pressing force, the icon is moved upward slower than that at the time of the significant depressurization as illustrated in Fig. 8D. This allows the user to intuitively understand a pressurization degree.

In a case of the instruction to maintain the current pressing force, both the upward and downward animations are stopped as illustrated in Fig. 9E. Alternatively, both the upward and downward animations are displayed as illustrated in Fig. 9F. Therefore, the user may intuitively understand that the instruction is not to pressurize or depressurize but to maintain the pressing force.

Note that, a moving speed of the icon is, for example, one cycle per 0.5 seconds in a case of the significant pressurization/depressurization, and one cycle per 1 second in a case of the modest pressurization/depressurization.

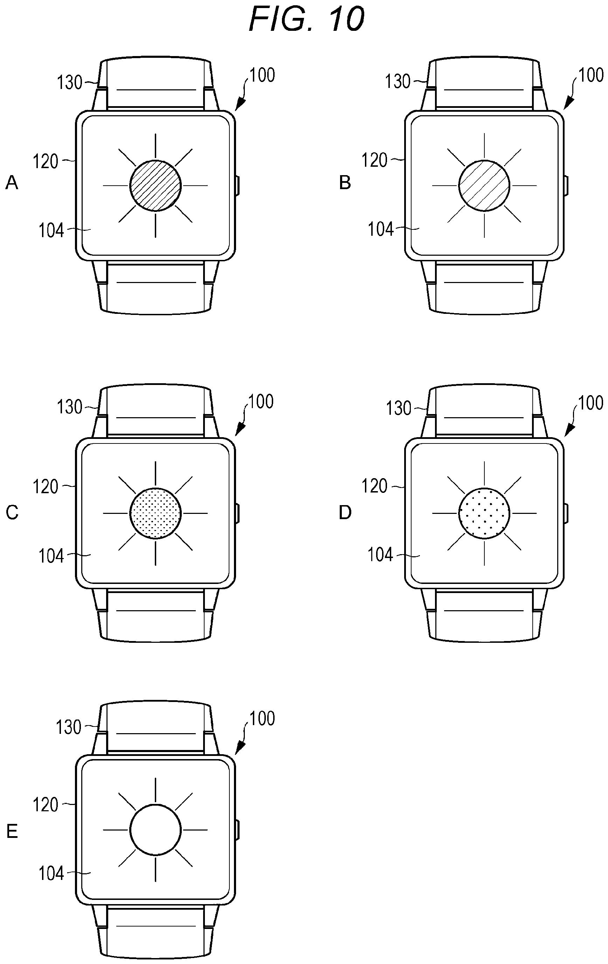

Next, a display of a colored icon in Fig. 10 is described.

As illustrated in Figs. 10A and 10B, an instruction to pressurize is issued by displaying an icon of a specific color. The specific color is, for example, red and the like. In a case of the instruction to significantly pressurize from the current pressing force, an icon in a dark color is displayed as illustrated in Fig. 10A. On the other hand, in a case of the instruction to modestly pressurize from the current pressing force, an icon in a lighter color than that in the case of the significant pressurization is displayed as illustrated in Fig. 10B. This allows the user to intuitively understand a pressurization degree.

As illustrated in Figs. 10C and 10D, an instruction to depressurize is issued by displaying an icon in a color different from the pressurizing icon, for example, a blue icon. In a case of the instruction to significantly depressurize from the current pressing force, an icon in dark blue is displayed as illustrated in Fig. 10C. On the other hand, in a case of the instruction to modestly depressurize from the current pressing force, an icon in a lighter blue than that in the case of the significant pressurization is displayed as illustrated in Fig. 10D. This allows the user to intuitively understand a pressurization degree.

In a case of the instruction to maintain the current pressing force, as illustrated in Fig. 10E, an icon in another color different from the color indicating pressurization or the color indicating depressurization, for example, in white is turned on. Therefore, the user may intuitively understand that the instruction is not to pressurize or depressurize but to maintain the pressing force.

Note that, it is described above that the display as the output unit 104 is allowed to display icons and animations; however, it is also possible to realize a similar instruction not by the display but by blinking and turning on of a light provided on the measuring device 100.

Furthermore, in a case where the output unit 104 is a speaker that outputs a sound, the instruction is issued by an electronic sound (beep sound and the like) that is intermittently emitted at regular intervals.

An instruction to pressurize is issued by outputting a high-frequency electronic sound. In a case of the instruction to significantly pressurize from the current pressing force, the electronic sound is emitted intermittently at short intervals. On the other hand, in a case of the instruction to modestly pressurize from the current pressing force, the electronic sound is intermittently emitted at intervals longer than that in the significant pressurization. This allows the user to intuitively understand a pressurization degree.

On the other hand, an instruction to depressurize is issued by outputting an electronic sound at a lower-frequency than that of the electronic sound of the instruction to pressurize. In a case of the instruction to significantly depressurize from the current pressing force, the electronic sound is emitted intermittently at short intervals. On the other hand, in a case of the instruction to modestly depressurize from the current pressing force, the electronic sound is intermittently emitted at intervals longer than that in the significant depressurization. This allows the user to intuitively understand a pressurization degree.

In a case of the instruction to maintain the current pressing force, an electronic sound at a lower-frequency than that of the electronic sound to instruct the pressurization and at a higher-frequency than that of the electronic sound to instruct the depressurization is intermittently emitted or continuously emitted during a predetermined time.

Note that, a method of outputting the instruction information is not limited to that described with reference to Figs. 8 to 10, and may be any method as long as the user may intuitively recognize the instruction information with this. For example, it is possible to display a message such as "please press harder", "please press a little harder", "please press softer", "please press a little softer", and the like on the display as the output unit 104, or output them as a sound from a speaker, earphones, headphones, and the like as the output unit 104.

The first embodiment is configured as described above. According to the first embodiment, the user may press a part of the body such as the arm with the pressing unit 101 of the measuring device 100, thereby easily performing the blood pressure measurement with a simple configuration. Furthermore, the user is instructed to adjust the pressing force not by the specific numerical value and the like but by the instruction to pressurize or depressurize from the current pressing force or to maintain the current pressing force, so that the user may intuitively adjust the pressing force.

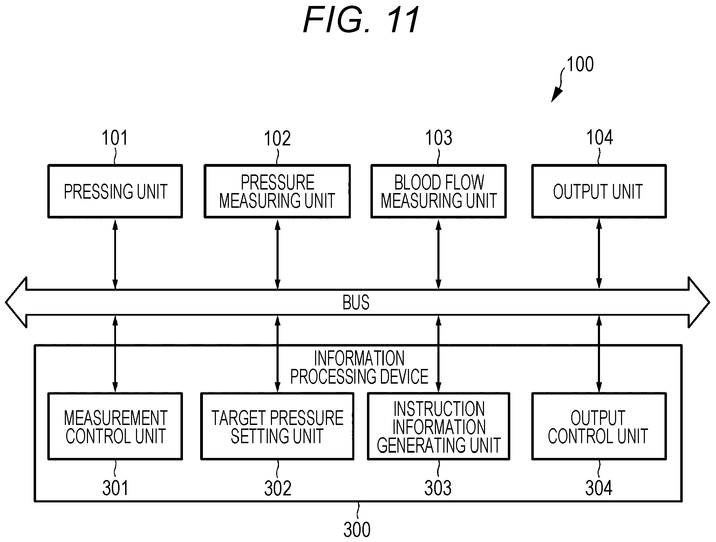

Note that, as illustrated in a block diagram in Fig. 11, a measurement control unit 301, a target pressure setting unit 302, an instruction information generating unit 303, and an output control unit 304 may form an information processing device 300. The information processing device 300 corresponds to an information processing device recited in claims.

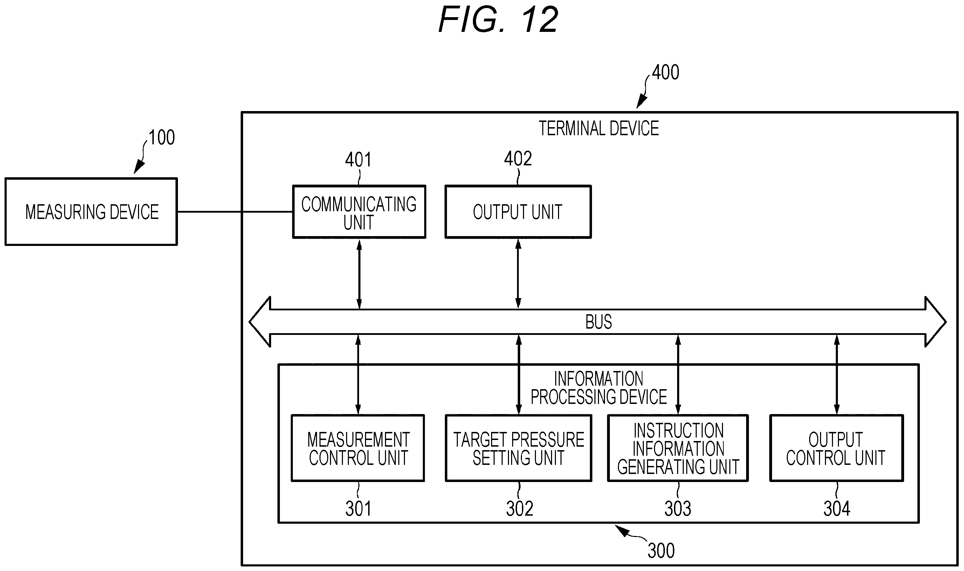

This information processing device 300 may operate in a terminal device 400 such as a smartphone, a tablet terminal, and a personal computer as illustrated in Fig. 12, in addition to a case where this functions in the measuring device 100 as illustrated in Fig. 11. In this case, a communicating unit 401 in the terminal device 400 and the measuring device 100 transmit and receive information such as the pressing force and the target pressure by wired or wireless communication, and the instruction information generating unit 303 of the information processing device 300 generates instruction information in the terminal device 400. Then, the output control unit 304 presents the instruction information to the user by outputting the instruction information by the output unit 402 of the terminal device 400. The output unit 402 of the terminal device 400 includes a display and a speaker provided on the terminal device 400, and a sound output processing unit and the like to supply a sound signal to earphones, headphones, and the like connected to the terminal device 400.

### <2. Second Embodiment>

### [2-1. Configuration of measuring device]

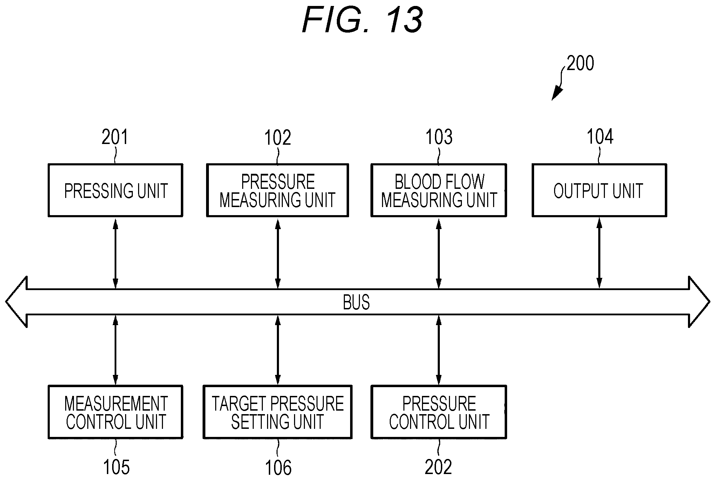

Next, a second embodiment of the present disclosure is described. A configuration of a measuring device 200 according to the second embodiment is described with reference to Fig. 13. In the second embodiment, a specific configuration of a pressing unit 201 is different from that of the pressing unit 101 in the first embodiment. Furthermore, the second embodiment is different from the first embodiment in including a pressing control unit 202 and not including an instruction information generating unit and an output control unit. Other configurations are similar to those in the first embodiment, so that they are not described. The second embodiment does not adjust a pressing force by presenting instruction information to a user but adjusts the pressing force under control in the measuring device 200.

The pressing control unit 202 controls to adjust a pressing force by controlling a pressing operation of the pressing unit 201 on the basis of a difference between a pressing force measured by a pressure measuring unit 102 and a target pressure set by a target pressure setting unit 106.

### [2-2. Configuration of pressing unit]

Next, a configuration of the pressing unit 201 is described. Note that, configuration examples of the pressing unit 201 include first to third examples, and in any example, the measuring device 200 includes a casing 120 and a band portion 130 and is a wearable device wound around a user's arm 1000 as illustrated in Fig. 2. Furthermore, in all the first to third examples, the pressure measuring unit 102 and a blood flow measuring unit 103 are arranged close to each other on a side brought into contact with the user's arm 1000 on an inner side of the measuring device 200.

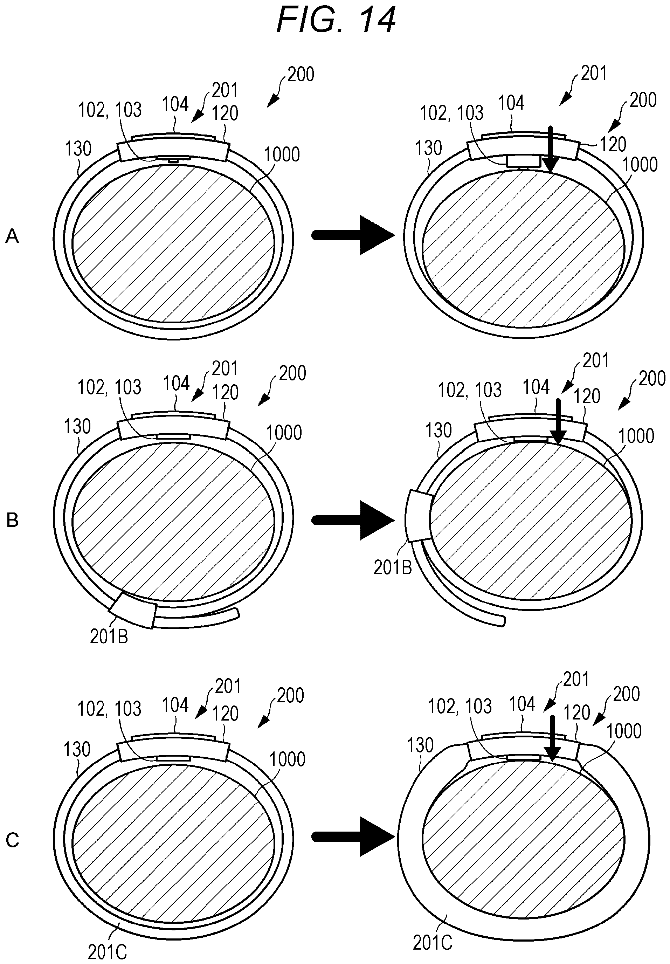

As illustrated in Fig. 14A, the first example is configured as an operating mechanism that presses the pressure measuring unit 102 and the blood flow measuring unit 103 in a direction toward the user's arm 1000 on a surface on a side of the user's arm 1000 of the casing 120. An operation of this operating mechanism is controlled by a pressing control unit 202. Therefore, the pressing unit 201 as the operating mechanism may press the pressure measuring unit 102 and the blood flow measuring unit 103 against the user's arm 1000 to eliminate a pulse wave and measure a blood pressure.

In the second example, the pressing unit 201 is configured as an automatic adjusting mechanism 201B that adjusts a band length of the band portion 130 of the measuring device 200 as illustrated in Fig. 14B. An operation of the automatic adjusting mechanism 201B is controlled by the pressing control unit 202, and by adjusting a tightening degree of the band portion 130, the pressure measuring unit 102 and the blood flow measuring unit 103 may press the user's arm 1000 to eliminate the pulse wave and measure the blood pressure. Examples of the automatic adjusting mechanism 201B include a mechanism of automatically winding the band portion 130 and the like.

Note that, a buckle mechanism used when attaching the band portion 130 and the automatic adjusting mechanism of the length of the band portion 130 may be integrally configured or may be separately configured.

In the third example, as illustrated in Fig. 14C, the pressing unit 201 is configured as an automatic adjusting mechanism 201C that adjusts the tightening degree of the arm 1000 by the band portion 130 by automatically adjusting a diameter (thickness) of the band portion 130. An operation of the automatic adjusting mechanism 201C is controlled by the pressing control unit 202, and by adjusting the tightening degree of the band portion 130, the pressure measuring unit 102 and the blood flow measuring unit 103 may press the user's arm 1000 to eliminate the pulse wave and measure the blood pressure. As a method of adjusting the diameter (thickness) of the band portion 130, for example, there is a method of making the inside of the band portion 130 a highly airtight cavity and supplying air to the inside of the band portion 130 by controlling a small pump (not illustrated) to inflate.

### [2-3. Pressing controlling process]

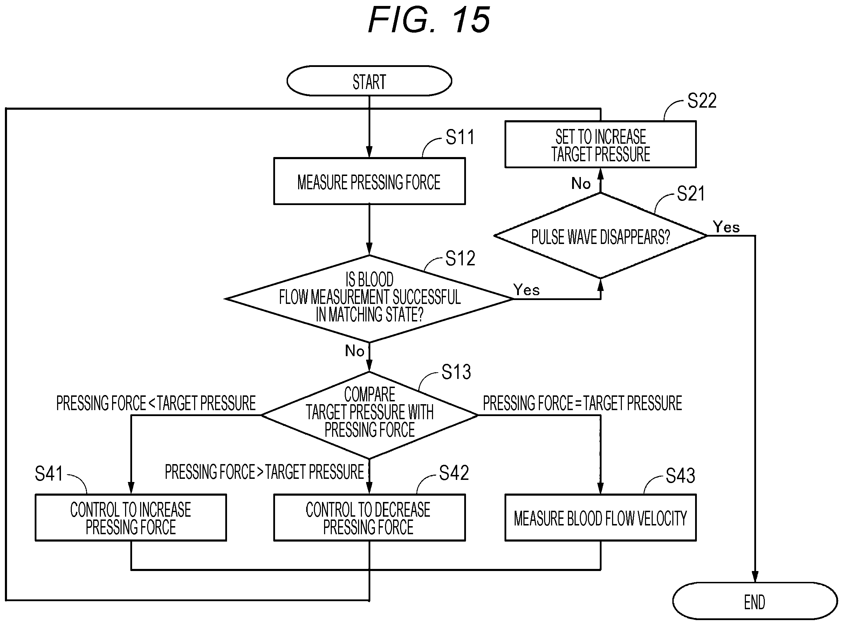

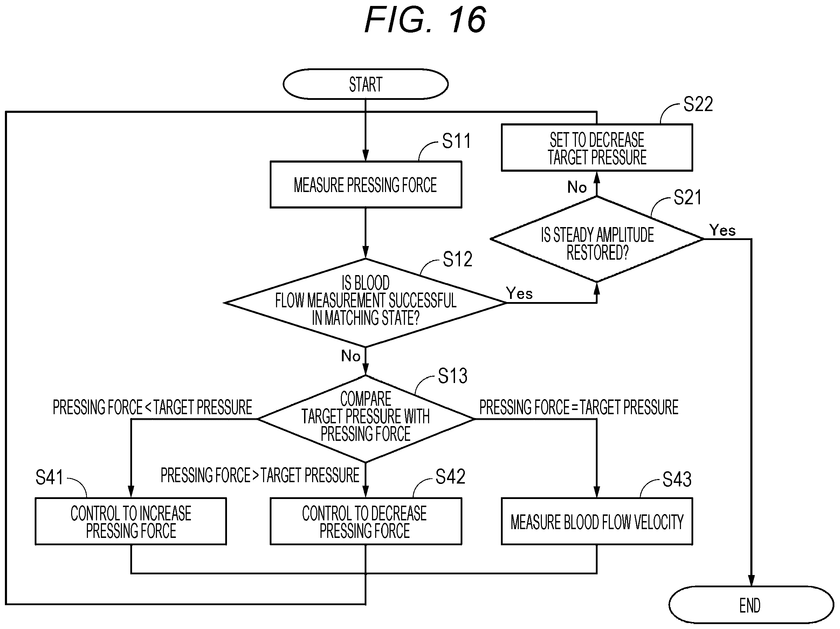

Next, a pressing force controlling process in the second embodiment is described with reference to flowcharts in Figs. 15 and 16. First, a process of adjusting a pressing force to obtain a maximum blood pressure is described with reference to Fig. 15. Note that, the same step numbers are assigned to a flow similar to that of the process in the first embodiment in Fig. 5, and description thereof is not repeated.

At step S13, the target pressure is compared with the current pressing force measured by the pressure measuring unit 102 at step S11, and in a case where the pressing force is smaller than the target pressure, the procedure shifts to step S41. Then, at step S41, the pressing control unit 202 controls the pressing unit 101 to increase the pressing force so as to match the target pressure.

Furthermore, in a case where the pressing force is larger than the target pressure in the comparison at step S13, the procedure shifts to step S42. Then, at step S42, the pressing control unit 202 controls the pressing unit 101 to decrease the pressing force so as to match the target pressure.

Moreover, in a case where the pressing force matches the target pressure in the comparison at step S13, the procedure shifts to step S43. Then, at step S43, the blood flow measuring unit 103 measures the blood flow velocity of the user.

As described above, a process in a case of increasing the pressing force to obtain the maximum blood pressure is performed.

The flowchart in Fig. 16 illustrates a process in a case of adjusting the pressing force to obtain a minimum blood pressure. In the flowchart in Fig. 16 also, similarly to the flowchart in Fig. 15, control to increase the pressing force is performed at step S41, control to decrease the pressing force is performed at step S42, and the measurement of the blood flow velocity is performed at step S43 on the basis of a comparison result between the pressing force and the target pressure at step S13.

The measuring device 200 according to the second embodiment is configured as described above. According to the second embodiment, the blood pressure may be easily measured by appropriately adjusting the pressing force.

### <3. Variation>

Although the embodiments of the present technology are heretofore described specifically, the present technology is not limited to the above-described embodiments, and various modifications based on the technical idea of the present technology may be made.

A measuring device 100 may be attached to a user's body, or may be put on a part of the user's body (for example, an arm) and press the part of the body to measure a blood flow velocity.

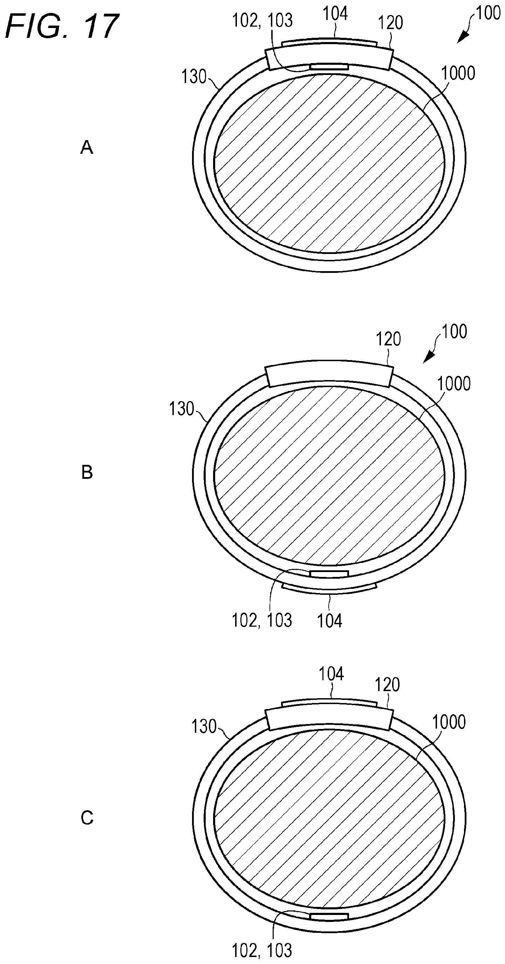

In the measuring device 100, arranging positions of a pressure measuring unit 102 and a blood flow measuring unit 103 and an arranging position of a display as an output unit 104 are not limited to one place. Fig. 17A illustrates an example in which the pressure measuring unit 102 and the blood flow measuring unit 103 are arranged between a casing 120 that forms the measuring device 100 and a user's arm 1000. In the example in Fig. 17A, an output unit 104 is provided on the casing 120.

Fig. 17B illustrates an example in which the pressure measuring unit 102 and the blood flow measuring unit 103 are arranged between the band portion 130 on the opposite side of the casing 120 that forms the measuring device 100 and the user's arm 1000. In the example in Fig. 17B, the output unit 104 is provided on the band portion 130.

Fig. 17C illustrates an example in which the pressure measuring unit 102 and the blood flow measuring unit 103 are arranged between the band portion 130 on the opposite side of the casing 120 that forms the measuring device 100 and the user's arm 1000. In the example in Fig. 17C, the output unit 104 is provided on the casing 120.

As illustrated in Figs. 17B and 17C, in a case where the pressure measuring unit 102 and the blood flow measuring unit 103 are provided on the band portion 130 not on the casing 120 side but on the opposite side of the casing 120, the pressure measuring unit 102 and the blood flow measuring unit 103 need to be connected to a measurement control unit 105 and the like provided in the casing 120 by a signal line passing through the band portion 130 or by wireless communication.

A variation of the arrangement of the pressure measuring unit 102, the blood flow measuring unit 103, and the output unit 104 illustrated in Fig. 17 may be combined with any configuration of the pressing unit 101 and the pressing unit 201 illustrated in Figs. 3A to 3C and Figs. 14A to 14C, respectively.

Furthermore, the present technology may be applied to any wearable device that may be worn on a body part capable of measuring a blood pressure. For example, there are a ring-type device to be worn on a finger, an earring-type device to be worn on an ear, an armband-type device to be worn on an arm, and the like.

The present technology may also have following configurations.
(1) A measuring device provided with:
   a pressing unit that presses a part of a user's body according to adjustment by a user;
   a pressure measuring unit that measures a pressing force by the pressing unit; and
   a blood flow measuring unit that measures a blood flow velocity of the user in a case where the pressing force is in a predetermined state.
(2) The measuring device according to (1), further provided with:
   an instruction information generating unit that generates instruction information to instruct the user to change an adjusting degree on the basis of a measurement result by the pressure measuring unit; and
   an output unit that outputs the instruction information.
(3) The measuring device according to (1) or (2), further provided with:
   a target pressure setting unit that sets a target pressure that is a target of the pressing force to press a part of the user's body.
(4) The measuring device according to (3),
   in which the predetermined state is a state in which the pressing force matches the target pressure.
(5) The measuring device according to (2),
   in which the instruction information is generated on the basis of a difference between the pressing force and the target pressure.
(6) The measuring device according to (5),
   in which, in a case where the pressing force is smaller than the target pressure, the instruction information is information to instruct the user to pressurize.
(7) The measuring device according to (6),
   configured to set a pressurization degree of the instruction information on the basis of comparison between the difference between the pressing force and the target pressure, and a threshold.
(8) The measuring device according to any one of (5) to (7),
   in which, in a case where the pressing force is larger than the target pressure, the instruction information is information to instruct the user to depressurize.
(9) The measuring device according to (8),
   configured to set a depressurization degree of the instruction information on the basis of comparison between the difference between the pressing force and the target pressure, and a threshold.
(10) The measuring device according to any one of (5) to (9),
   in which, in a case where the pressing force is equal to the target pressure, the instruction information is information to instruct the user to maintain the pressing force.
(11) The measuring device according to any one of (2) to (10),
   in which the instruction information is output by the output unit as at least any one of light blinking, a moving image, a color change, a sound, or a character.
(12) A measuring method provided with:
   pressing a part of a user's body by a pressing unit according to adjustment by a user;
   measuring a pressing force by the pressing unit; and
   measuring a blood flow velocity of the user in a case where the pressing force is in a predetermined state.
(13) A measuring device provided with:
   a pressing unit that presses a part of a user's body;
   a pressure measuring unit that measures a pressing force by the pressing unit;
   a blood flow measuring unit that measures a blood flow velocity of a user in a case where the pressing force is in a predetermined state;
   a target pressure setting unit that sets a target pressure being a target of the pressing force; and
   a pressing control unit that controls the pressing unit such that the pressing force matches the target pressure.
(14) The measuring device according to (13),
   in which the predetermined state is a state in which the pressing force matches the target pressure.
(15) The measuring device according to (13) or (14),
   in which pressing by the pressing unit includes at least any one of diameter adjustment of a band portion or pressing in a direction toward the user.
(16) A measuring method provided with:
   pressing a part of a user's body to measure a pressing force;
   measuring a blood flow velocity of a user in a case where the pressing force is in a predetermined state;
   setting a target pressure being a target of the pressing force; and
   controlling the pressing unit such that the pressing force matches the target pressure.

### REFERENCE SIGNS LIST

100, 200 Measuring device
101 Pressing unit
102 Pressure measuring unit
103 Blood flow measuring unit
104 Output unit
106 Target pressure setting unit
107 Instruction information generating unit
108 Output control unit

## Claims

1. A measuring device comprising:
a pressing unit that presses a part of a user's body according to adjustment by a user;
a pressure measuring unit that measures a pressing force by the pressing unit; and
a blood flow measuring unit that measures a blood flow velocity of the user in a case where the pressing force is in a predetermined state.

2. The measuring device according to claim 1, further comprising:
an instruction information generating unit that generates instruction information to instruct the user to change an adjusting degree on a basis of a measurement result by the pressure measuring unit; and
an output unit that outputs the instruction information.

3. The measuring device according to claim 1, further comprising:
a target pressure setting unit that sets a target pressure that is a target of the pressing force to press a part of the user's body.

4. The measuring device according to claim 3,
wherein the predetermined state is a state in which the pressing force matches the target pressure.

5. The measuring device according to claim 2,
wherein the instruction information is generated on a basis of a difference between the pressing force and the target pressure.

6. The measuring device according to claim 5,
wherein, in a case where the pressing force is smaller than the target pressure, the instruction information is information to instruct the user to pressurize.

7. The measuring device according to claim 6,
configured to set a pressurization degree of the instruction information on a basis of comparison between the difference between the pressing force and the target pressure, and a threshold.

8. The measuring device according to claim 5,
wherein, in a case where the pressing force is larger than the target pressure, the instruction information is information to instruct the user to depressurize.

9. The measuring device according to claim 8,
configured to set a depressurization degree of the instruction information on a basis of comparison between the difference between the pressing force and the target pressure, and a threshold.

10. The measuring device according to claim 5,
wherein, in a case where the pressing force is equal to the target pressure, the instruction information is information to instruct the user to maintain the pressing force.

11. The measuring device according to claim 2,
wherein the instruction information is output by the output unit as at least any one of light blinking, a moving image, a color change, a sound, or a character.

12. A measuring method comprising:
pressing a part of a user's body by a pressing unit according to adjustment by a user;
measuring a pressing force by the pressing unit; and
measuring a blood flow velocity of the user in a case where the pressing force is in a predetermined state.

13. A measuring device comprising:
a pressing unit that presses a part of a user's body;
a pressure measuring unit that measures a pressing force by the pressing unit;
a blood flow measuring unit that measures a blood flow velocity of a user in a case where the pressing force is in a predetermined state;
a target pressure setting unit that sets a target pressure being a target of the pressing force; and
a pressing control unit that controls the pressing unit such that the pressing force matches the target pressure.

14. The measuring device according to claim 13,
wherein the predetermined state is a state in which the pressing force matches the target pressure.

15. The measuring device according to claim 13,
wherein pressing by the pressing unit includes at least any one of diameter adjustment of a band portion or pressing in a direction toward the user.

16. A measuring method comprising:
pressing a part of a user's body to measure a pressing force;
measuring a blood flow velocity of a user in a case where the pressing force is in a predetermined state;
setting a target pressure being a target of the pressing force; and
controlling the pressing unit such that the pressing force matches the target pressure.
